# EUROPÄISCHE PATENTANMELDUNG

(11) **EP 4 035 613 A1**
(43) Veröffentlichungstag der Anmeldung: **03.08.2022**
(21) Anmeldenummer: 21211040.7
(22) Anmeldetag: 29.11.2021
(51) Int. Cl.: A61B 17/32, A61B 17/00, H01H 25/00, A61B 17/16

(54) **MEDIZINISCHES INSTRUMENT MIT VERBESSERTER HANDHABUNG**

(30) Priorität: 28.01.2021 DE 102021101897
(71) Anmelder: Eberle GmbH & Co. KG, 75449 Wurmberg (DE)
(72) Erfinder: LÖFFLER, Heiko, 75449 Wurmberg (DE)
(74) Vertreter: Hoefer & Partner Patentanwälte mbB

(57) **Zusammenfassung**

Die Erfindung betrifft ein medizinisches Instrument für arthroskopische Eingriffe, umfassend ein Handstück (2), ein Schneidenhalter (3) mit einem äußeren Hohlrohr (30) und einem inneren hohlen Schneidenrohr (31) mit Schneiden (32) an einem distalen Ende des Innenrohrs, und eine Verbindungsanordnung (4) zum lösbaren Verbinden des Handstücks (2) mit dem Schneidenhalter (3), wobei die Verbindungsanordnung (4) zwei sich in Axialrichtung (X-X) erstreckende Arme (40) am Schneidenhalter (3) aufweist, die sich einander gegenüberliegen und an ihren freien Enden ein Rastelement (41) aufweist, wobei die Arme (40) ferner jeweils einen Drückbereich (42) und einen elastischen Bereich (43) aufweisen, wobei das Handstück (2) an einem Innenumfang eine Aufnahme (44) mit einer Hinterschneidung (44a) für die Rastelemente (41) aufweist, und wobei das Handstück ferner an einem distalen Endbereich zwei Ausnehmungen (45) zur teilweisen Aufnahme der Drückbereiche (42) aufweist.

## Beschreibung

Die vorliegende Erfindung betrifft ein medizinisches Instrument, insbesondere für arthroskopische Eingriffe in Knie oder Hüfte oder Sprunggelenk, welches eine vereinfachte Handhabung ermöglicht.

Medizinische Instrumente für arthroskopische Eingriffe sind aus dem Stand der Technik in unterschiedlichen Ausgestaltungsformen bekannt. Das medizinische Instrument umfasst üblicherweise ein Handstück und einen Schneidenhalter. Die Handstücke sind dabei mit einem Antrieb verbunden oder weisen einen Antrieb auf, so dass Schneideninstrumente im Schneidenhalter rotativ angetrieben werden können, um Gewebe oder anderes biologisches Material insbesondere aus einem Gelenkbereich zu entfernen. Üblicherweise weisen derartige medizinische Instrumente ferner noch eine Absaugeinrichtung zum Absaugen von abgetrenntem Material auf, welche durch das medizinische Instrument hindurchgeführt wird. Weiterhin ist der Schneidenhalter lösbar am Handstück angebracht, damit eine entsprechende Reinigung der Teile des medizinischen Instruments möglich ist. Hierbei sollte das Lösen und Verbinden von Handstück und Schneidenhalter möglichst schnell, einfach und sicher sein. Eine derartige Verbindungsanordnung ist hierbei beispielsweise aus der EP 1 790 292 A1 bekannt. Diese Verbindungsanordnung umfasst jedoch relativ viele Teile und ist nach einem Gebrauch relativ aufwendig zu reinigen.

Es ist daher Aufgabe der vorliegenden Erfindung, ein medizinisches Instrument bereitzustellen, welches bei einfachem Aufbau und einfacher, kostengünstiger Herstellbarkeit ein schnelles und sicheres Lösen und Verbinden zwischen Handstück und Schneidenhalter ermöglicht sowie eine besonders einfache und schnelle Reinigung möglich macht.

Diese Aufgabe wird durch ein medizinisches Instrument mit den Merkmalen des Anspruchs 1 gelöst. Die Unteransprüche zeigen bevorzugte Weiterbildungen der Erfindung.

Das medizinische Instrument für arthroskopische Eingriffe mit den Merkmalen des Anspruchs 1 weist demgegenüber den Vorteil auf, dass ein schnelles und einfaches Lösen eines Schneidenhalters von einem Handstück sowie ein schnelles Verbinden des Schneidenhalters mit dem Handstück möglich ist. Weiterhin können sowohl das Handstück als auch der Schneidenhalter sehr einfach und gründlich in kurzer Zeit gereinigt werden. Dies wird erfindungsgemäß dadurch erreicht, dass das medizinische Instrument eine Verbindungsanordnung zum lösbaren Verbinden des Handstücks mit dem Schneidenhalter aufweist. Der Schneidenhalter umfasst dabei ein Außenrohr und ein Innenrohr mit Schneiden am distalen Ende. Die Verbindungsanordnung weist zwei sich in Axialrichtung des medizinischen Instruments erstreckende Arme auf, die am Schneidenhalter angeordnet sind. Die Arme liegen sich einander gegenüber und weisen an ihren freien Enden ein Rastelement auf. Ferner weisen die Arme jeweils einen Drückbereich zur Betätigung und einen elastischen Bereich auf. Das Handstück weist an einem Innenumfang eine Aufnahme mit einer Hinterschneidung für die Rastelemente auf. Darüber hinaus weist das Handstück an einem distalen Endbereich zwei Ausnehmungen auf, welche zur teilweisen Aufnahme der Arme dienen. Die Rastelemente und die Hinterschneidung stellen eine axiale Sicherung des Schneidenhalters am Handstück bereit. Die zwei Ausnehmungen am distalen Endbereich des Handstücks und die in den Ausnehmungen angeordneten Bereiche der Arme stellen eine Verdrehsicherung bereit. Somit weist die erfindungsgemäße Verbindungsanordnung zwischen dem Handstück und dem Schneidenhalter neben der axialen Sicherung gleichzeitig auch eine Verdrehsicherung auf. Dabei ist eine Teileanzahl der Verbindungsanordnung sehr klein. Weiterhin können die Teile der Verbindungsanordnung schnell und einfach gereinigt werden. Dadurch ist die Verbindungsanordnung insbesondere geeignet, bei mehrfach verwendbaren Schneidenhaltern eingesetzt zu werden. Da die Verbindungsanordnung auch relativ kostengünstig herstellbar ist, beispielsweise aus einem Kunststoff, ist die Verbindungsanordnung jedoch auch geeignet, bei Einmal-Instrumenten verwendet zu werden.

Vorzugsweise stehen die Drückbereiche von den Armen radial nach außen vor. Die Drückbereiche stehen dabei besonders bevorzugt von einem Außenumfang des Handstücks im Bereich der Verbindungsanordnung nach außen vor, so dass eine einfache Betätigung, insbesondere zum Lösen des Schneidenhalters vom Handstück, ermöglicht wird.

Weiter bevorzugt weisen die Rastelemente an den Armen eine Rampe auf. Die Rampe weist vorzugsweise einen Winkel von ≤ 45° auf. Dadurch kann eine besonders einfache Verbindung durch eine in Axialrichtung erfolgte Relativverschiebung zwischen dem Schneidenhalter und dem Handstück erfolgen, so dass der Schneidenhalter einfach auf das Handstück aufgesetzt werden kann und in Axialrichtung bewegt werden kann. Die Arme gleiten dann an den Rampen in einem Innenbereich des Handstücks und rasten federnd hinter der Hinterschneidung am Handstück ein.

Weiter bevorzugt sind die Arme einstückig mit einem Ring ausgebildet, wobei sich der elastische Bereich der Arme an einem Bereich zwischen dem Ring und den Drückbereichen befindet. Dadurch kann eine Befestigung der Verbindungsanordnung mittels des Rings am Schneidenhalter erfolgen und die Arme müssen lediglich die Funktionen der Verdrehsicherung und der axialen Sicherung übernehmen.

Der Ring, an welchem die Arme befestigt sind, ist vorzugsweise lösbar mittels einer Clipsverbindung mit dem Schneidenhalter verbunden. Dadurch ist es auch möglich, dass der Ring mitsamt den Armen vom Schneidenhalter gelöst werden kann und separat eine Reinigung von Schneidenhalter und diesem Teil der Verbindungsanordnung mit Ring und Armen möglich ist.

Die Ausnehmungen im Handstück zur Aufnahme von Teilbereichen der Arme sind vorzugsweise U-förmig ausgebildet. Dabei weisen die Arme entsprechend den Ausnehmungen U-förmige Bereiche, insbesondere an den Drückbereichen der Arme, auf.

Weiter bevorzugt weist das Handstück eine Tastenanordnung zur Betätigung des medizinischen Instruments auf. Die Tastenanordnung ist dabei nahe dem distalen Ende des Handstücks angeordnet, so dass die Tastenanordnung nahe am Schneidenhalter liegt. Die Tastenordnung ist in Umfangsrichtung zwischen den Ausnehmungen für die Aufnahme der Arme der Verbindungsanordnung angeordnet. Dadurch kann ein besonders kompakter Aufbau des medizinischen Instruments, insbesondere in Axialrichtung ermöglicht werden.

Die Tastenanordnung umfasst vorzugsweise eine erste, zweite und dritte Taste sowie einen ersten und einen zweiten Tastkontakt. Dabei sind die drei Tasten relativ zu den beiden Tastkontakten derart angeordnet, dass die erste Taste nur den ersten Tastkontakt betätigt, die zweite Taste nur den zweiten Tastkontakt betätigt und die dritte Taste gleichzeitig den ersten und zweiten Tastkontakt betätigt. Durch diesen Aufbau der Tastenanordnung kann eine große bauliche Vereinfachung der Tastenanordnung ermöglicht werden. Dabei können zwei Tastkontakte durch drei Tasten unterschiedlich betätigt werden, so dass drei Betriebszustände des medizinischen Instruments mit nur zwei Tastkontakten möglich sind. Beispielsweise ist die erste Taste für einen Rechtslauf des medizinischen Instruments eingerichtet, die zweite Taste für einen Linkslauf des medizinischen Instruments eingerichtet und die dritte Taste für eine oszillierende Bewegung des medizinischen Instruments in Links-Rechts-Richtung eingerichtet.

Die drei Tasten der Tastenanordnung sind vorzugsweise in Reihe in Axialrichtung des medizinischen Instruments derart angeordnet, dass die dritte Taste zwischen der ersten und zweiten Taste angeordnet ist. Somit kann auf einfache Weise eine mechanische Umsetzung bei Betätigung der dritten Taste zur Kontaktierung des ersten und zweiten Tastkontaktes gleichzeitig ermöglicht werden. Die drei Tasten sind dabei auf einer gemeinsamen, starren Tastenplatte angeordnet. Die starre Tastenplatte liegt dabei über dem ersten und zweiten Tastkontakt. Die starre Tastenplatte ist vorzugsweise um eine Kippachse, die senkrecht zur Mittelachse X-X des medizinischen Instruments ist, kippbar.

Die Tastenplatte ist vorzugsweise aus Metall hergestellt. Weiter bevorzugt ist eine Steuerplatine in die Tastenanordnung integriert.

Weiter bevorzugt umfasst das medizinische Instrument ferner eine Absaugungsregulierung, welche am Handstück in Reihe mit der Tastenanordnung angeordnet ist. Dadurch kann das medizinische Instrument in einem Einhand-Betrieb verwendet werden, wobei gleichzeitig das Schneideninstrument in entsprechender Richtung betätigt werden kann und eine Absaugung von abgetrenntem Gewebe reguliert werden kann.

Das medizinische Instrument wird vorzugsweise bei Gelenk-Operationen oder Meniskus-Operationen oder anderen endoskopischen Eingriffen verwendet.

Nachfolgend wird ein bevorzugtes Ausführungsbeispiel der Erfindung unter Bezugnahme auf die begleitende Zeichnung im Detail beschrieben. In der Zeichnung ist:
- Fig. 1: eine schematische, perspektivische Ansicht eines medizinischen Instruments gemäß einem bevorzugten Ausführungsbeispiel der Erfindung,
- Fig. 2: eine schematische Schnittansicht durch ein Handstück des medizinischen Instruments von Fig. 1,
- Fig. 3: eine schematische Ansicht des Schneidenhalters mit einem Teil der Verbindungsanordnung zur Verbindung mit dem Handstück,
- Fig. 4: eine schematische, perspektivische Ansicht des am Schneidenhalter angeordneten Teils der Verbindungsanordnung, und
- Fig. 5: eine schematische, perspektivische Explosionsdarstellung einer Tastenanordnung des medizinischen Instruments von Fig. 1.

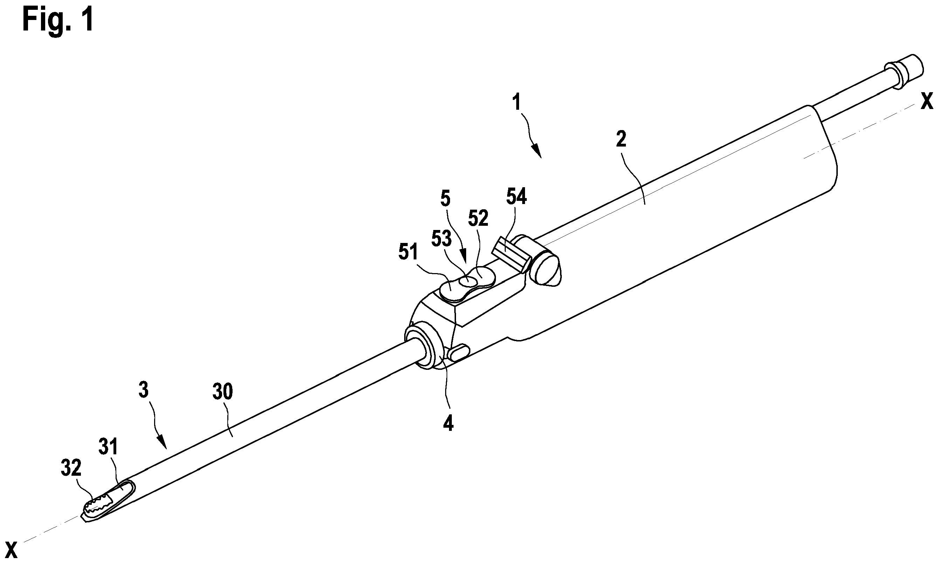

Nachfolgend wird unter Bezugnahme auf die Fig. 1 bis 5 ein medizinisches Instrument 1 für arthroskopische Eingriffe gemäß einem ersten bevorzugten Ausführungsbeispiel der Erfindung im Detail beschrieben.

Wie aus Fig. 1 ersichtlich ist, umfasst das medizinische Instrument 1 ein Handstück 2 und einen Schneidenhalter 3.

Im Handstück 2 ist beispielsweise im Hohlraum 22 ein Antrieb angeordnet oder alternativ ist das Handstück mit einem Antrieb verbindbar.

Der Schneidenhalter 3 umfasst ein äußeres Hohlrohr 30 und ein inneres Schneidenrohr 31 mit Schneiden 32 an einem distalen Ende.

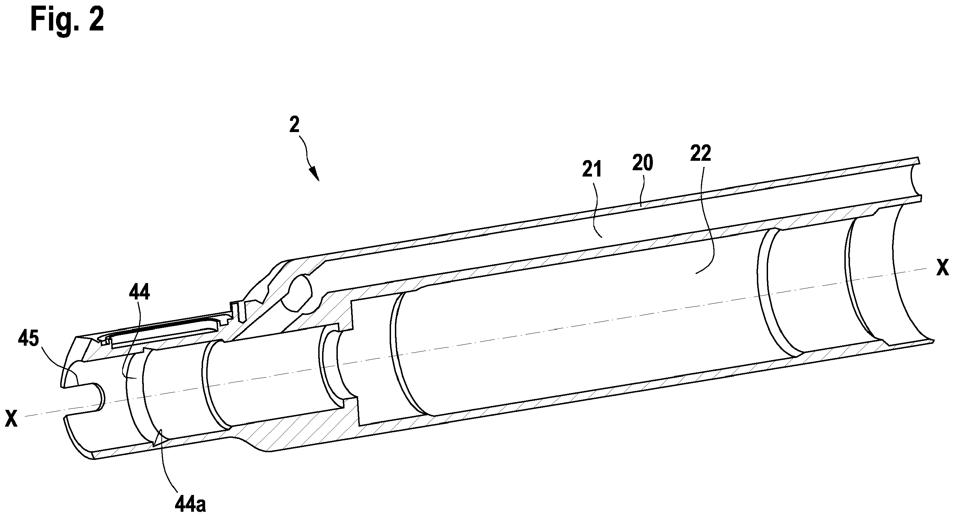

Am Schneidenhalter 3 ist ferner ein Teil einer Verbindungsanordnung 4 angeordnet. Die Verbindungsanordnung 4 ist im Detail aus den Fig. 2 bis 4 ersichtlich.

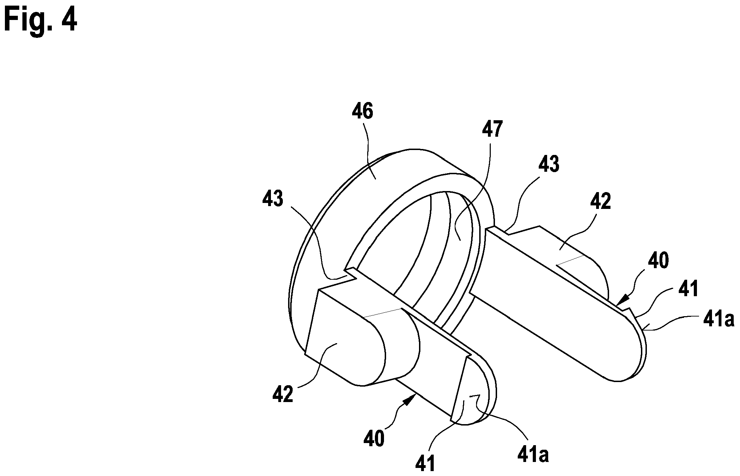

Wie insbesondere aus Fig. 4 ersichtlich ist, umfasst der Teil der Verbindungsanordnung 4, welcher am Schneidenhalter 3 angeordnet ist, einen Ring 46, von welchem zwei Arme 40 in Axialrichtung X-X des medizinischen Instruments vorstehen. Die beiden Arme 40 liegen sich am Ring 46 einander gegenüber. Der Ring 46 umfasst eine Clips-Verbindung 47, mit welcher der Ring an den Schneidenhalter 3 angeclipst werden kann. Dadurch ist der Ring 46 lösbar am Schneidenhalter 3 angeordnet.

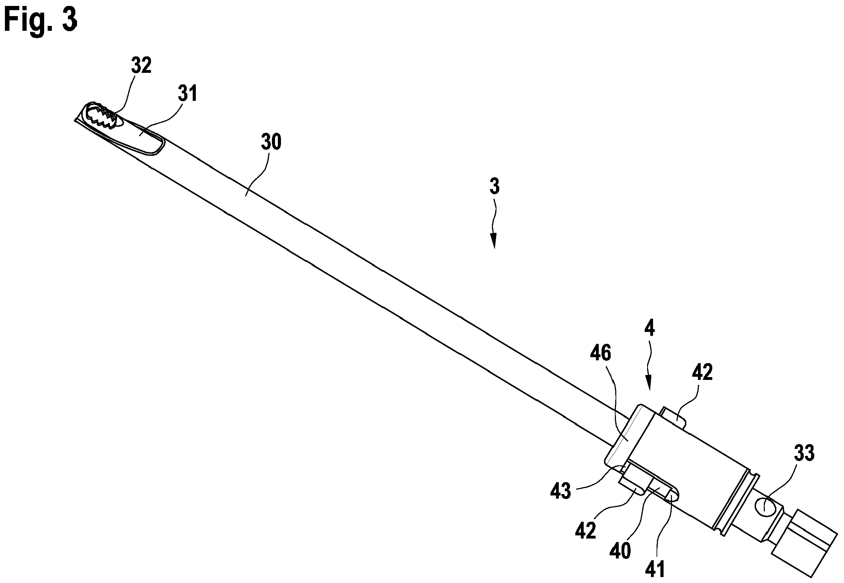

Die beiden Arme 40 sind gleich aufgebaut und weisen ein Rastelement 41 an einem distalen Ende, einen Drückbereich 42 zur Betätigung und einen elastischen Bereich 43 auf. Der elastische Bereich 43 ist dabei in Axialrichtung X-X zwischen dem Drückbereich und dem Ring 46 angeordnet. Der Drückbereich 42 ist Vollkörperbereich, welcher radial an den Armen 40 nach außen vorsteht. Wie insbesondere aus den Fig. 1 und 3 deutlich wird, stehen die Drückbereiche 43 somit seitlich vom medizinischen Instrument 1 vor. Dadurch ist ein einfaches Greifen und Betätigen der Drückbereiche zum Lösen des Schneidenhalters 3 vom Handstück 2 möglich.

Wie weiter aus Fig. 2 ersichtlich ist, umfasst die Verbindungsanordnung 4 im Handstück 2 in einem hohlen Handstückkörper 20 ferner eine Aufnahme 44 mit einer Hinterschneidung 44a. Die Hinterschneidung 44a verläuft als nutförmige Vertiefung entlang des Innenumfangs des hohlen Handstücks und kommt im montierten Zustand des Schneidenhalters 2 am Handstück 2 mit den Rastelementen 41 in Kontakt. Die Rastelemente 41 weisen dabei jeweils Rampen 41a auf, so dass durch einfaches axiales Aufschieben des Schneidenhalters 3 auf das Handstück 2 eine Verbindung hergestellt werden kann.

Dabei sind im Handstückkörper 20 ferner noch zwei U-förmige Ausnehmungen 45 ausgebildet. Die Ausnehmungen 45 dienen dabei zur Aufnahme der Drückbereiche 42. Dabei dienen die zwei Ausnehmungen 45 auch als Führungsbereich während des Aufschiebvorgangs des Schneidenhalters 3 auf das Handstück 2.

Im montierten Zustand des Schneidenhalters 3 am Handstück 2 sind somit die Druckbereiche 42 in den Ausnehmungen 45 aufgenommen und stehen radial nach außen über das Handstück 2 vor. Dadurch bilden die Drückbereiche 42 in Verbindung mit den Ausnehmungen 45 eine Verdrehsicherung des Schneidenhalters 3 gegenüber dem Handstück 2. Die Rastelemente 41, welche an der Hinterschneidung 44a eingerastet sind, bilden dabei eine axiale Sicherung des Schneidenhalters 3 am Handstück 2.

Somit kann eine verdrehsichere Verbindung zwischen dem Schneidenhalter 3 und dem Handstück 2 realisiert werden. Da die Verbindungsanordnung 4 auch vom Schneidenhalter 3 entfernbar ist, kann auch eine besonders einfache Reinigung der Teile des medizinischen Instruments ermöglicht werden.

Dabei ist die Verbindungsanordnung 4, die am Schneidenhalter 3 angeordnet ist, vorzugsweise ein Kunststoffteil. Durch das Vorstehen der Drückbereiche 42 von den Armen 40 kann auch eine einfache und sichere Betätigung durch einen Nutzer des medizinischen Instruments erfolgen.

Im Handstückkörper 20 ist ferner noch ein Absaugkanal 21 vorgesehen, über den abgesaugtes Gewebe, welches aus dem Schneidenhalter 3 aus einer Austrittsöffnung 33 austritt, abgeführt werden kann.

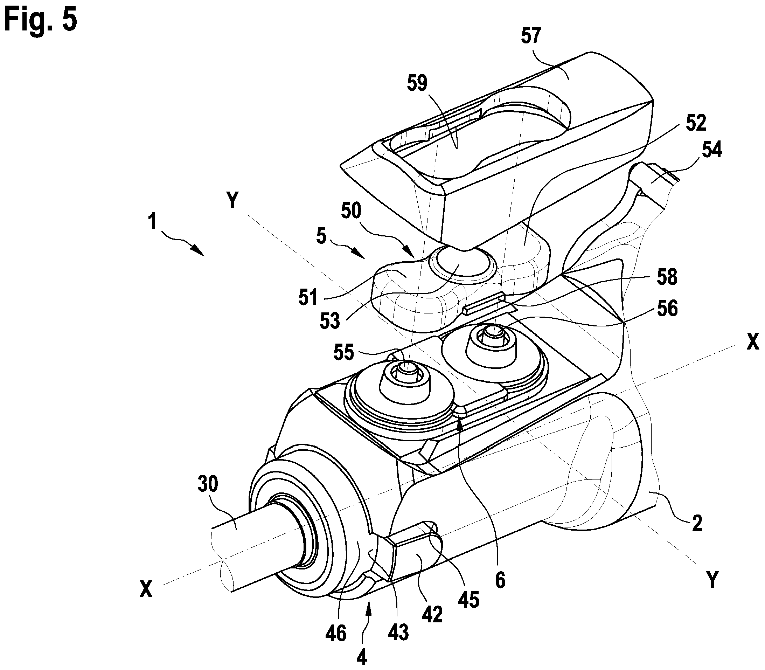

Am Handstück 2 ist ferner eine Tastenanordnung 5 zur Betätigung des medizinischen Instruments angeordnet. Die Tastenanordnung 5 umfasst eine erste Taste 51, eine zweite Taste 52 und eine dritte Taste 53. Wie aus Fig. 1 und 5 ersichtlich ist, sind die drei Tasten 51, 52, 53 in Axialrichtung X-X des medizinischen Instruments in Reihe angeordnet. Dabei ist die Tastenanordnung 5 an einem distalen Ende des Handstücks 2 nahe am Übergang zum Schneidenhalter 3 positioniert.

Die erste, zweite und dritte Taste 51, 52, 53 sind dabei auf einer starren Tastenplatte 50, vorzugsweise aus Edelstahl, integriert. Wie aus Fig. 5 ersichtlich ist, ist die Tastenplatte 50 länglich ausgebildet und liegt über einem ersten Tastkontakt 55 und einem zweiten Tastkontakt 56. Zwischen den beiden Tastkontakten 55, 56 befindet sich eine Kippachse Y-Y der Tastenanordnung. Die Kippachse Y-Y ist senkrecht zur Axialrichtung X-X des medizinischen Instruments 1.

Die Tastenanordnung 5 umfasst ferner einen Deckel 57, welcher teilweise die Tastenplatte und insbesondere die Tastenkontakte 55, 56 vor äußeren Einflüssen schützt. Wie aus Fig. 5 ersichtlich ist, sind im Deckel 57 zwei seitliche Aussparungen 59 vorgesehen, welche in Axialrichtung X-X verlaufen. An der Tastenplatte 50 sind zwei ebenfalls in Axialrichtung X-X verlaufende seitliche Vorsprünge 58 vorgesehen, welche im montierten Zustand in die seitlichen Aussparungen 59 im Deckel 57 eingreifen. Dadurch ist die Tastenplatte 50 in Vertikalrichtung senkrecht zur Axialrichtung X-X und auch in Axialrichtung X-X gesichert.

Weiterhin ist unter dem ersten und zweiten Tastkontakt 55, 56 eine Steuerplatine 6 in die Tastenanordnung 5 integriert. Dadurch ist es möglich, dass durch Betätigung des ersten und/oder zweiten Tastkontakts 55, 56 direkt elektronische Steuerelemente auf der Steuerplatine 6 aktiviert werden können, um das medizinische Instrument 1 zu steuern.

Die Tastenanordnung 5 umfasst somit nur genau die zwei Tastkontakte 55, 56. Der erste Tastkontakt 55 ist dabei unterhalb der ersten Taste 51 angeordnet und der zweite Tastkontakt 56 ist unterhalb der zweiten Taste 52 angeordnet. Somit kann durch Betätigung der ersten Taste 51 der erste Tastkontakt 55 aktiviert werden und durch Betätigung der zweiten Taste 52 der zweite Tastkontakt 56 aktiviert werden. Wie aus Fig. 1 und 5 ersichtlich ist, ist die dritte Taste 53 zwischen der ersten Taste 51 und der zweiten Taste 52 angeordnet. Die Tasteinrichtung 5 ist nun derart eingerichtet, dass durch Betätigung der dritten Taste 53 beide Tastkontakte 55, 56 gemeinsam mittels der starren Tastenplatte 50 betätigt werden.

Somit kann die Tastanordnung 5 mit drei Tasten 51, 52, 53 und zwei Tastkontakten 55, 56 drei unterschiedliche Betriebsmodi des medizinischen Instruments 1 bereitstellen. Beispielsweise kann die erste Taste einen Linkslauf des inneren Schneidenrohrs bewirken, die zweite Taste einen Rechtslauf des inneren Schneidenrohrs bewirken und die dritte Taste eine oszillierende Hin- und Herbewegung des inneren Schneidenrohrs ermöglichen.

Die Tastenanordnung 5 umfasst weiter bevorzugt zwei Rückstellelemente, welche vorzugsweise als Schnappscheiben ausgebildet sind. Dabei ist jeweils eine Schnappscheibe unter dem ersten Tastkontakt 55 bzw. unter dem zweiten Tastkontakt 56 angeordnet. Die beiden federnden Schnappscheiben stellen dabei nach einer Betätigung der Tasten die Tastenplatte 50 wieder in die Ausgangsposition zurück, indem die Schnappscheiben wieder ihre ursprüngliche Ausgangsposition einnehmen.

Weiterhin umfasst das Handstück 2 eine Absaugregulierung 54, welche, wie in Fig. 1 gezeigt, in Reihe nach der Tastenanordnung 5 angeordnet ist. Dadurch kann ein Nutzer des medizinischen Instruments üblicherweise mit einer Hand bzw. einem Finger die Tasten der Tastanordnung 5 sowie die Absaugregulierung 54 zur Regulierung der Absaugstärke des medizinischen Instruments betätigen.

Somit kann ein medizinisches Instrument 1 bereitgestellt werden, welches eine Verbindungsanordnung 4 aufweist, die eine sehr einfache Verbindung zwischen Schneidenhalter 3 und Handstück 2 sowie ein sehr einfaches Lösen und Befestigen des Schneidenhalters 3 vom/am Handstück 2 ermöglicht. Durch die über das Gehäuse des Handstücks 2 herausstehenden Drückbereiche 42 der Verbindungsanordnung 4 kann auch auf visuelle Weise ein einfaches Betätigen zum Lösen des Schneidenhalters 3 vom Handstück 4 ermöglicht werden.

Weiterhin kann durch das Vorsehen von zwei Armen 40, welche einander gegenüberliegend am Handstück 2 angeordnet sind, ein unbeabsichtigtes Lösen des Schneidenhalters 3 vom Handstück 2 verhindert werden. Dies ist insbesondere bei einer Verwendung des medizinischen Instruments 1 wichtig, da ein Nutzer üblicherweise das Handstück im Bereich der Tastenanordnung 4 und somit nahe an der Verbindungsanordnung 4 zwischen Handstück 2 und Schneidenhalter 3 hält. Dadurch kann nicht vollständig ausgeschlossen werden, dass der Nutzer unabsichtlich mit einem Finger auf den Drückbereich 42 drückt. Durch das Vorsehen von zwei einander gegenüberliegenden Drückbereichen 42 an dem Handstück 2 ist jedoch selbst in dem unbeabsichtigten Fall, dass der Nutzer aus Versehen auf einen Drückbereich 42 drückt, der Schneidenhalter 3 trotzdem noch sicher am Handstück 2 fixiert. Damit kann während einer Operation, wenn sich der Schneidenhalter 3 innerhalb eines Körperteils eines Patienten befindet, ein unbeabsichtigtes Lösen des Schneidenhalters 3 vom Handstück 2 verhindert werden.

Das Verbinden des Schneidenhalters 3 mit dem Handstück 4 kann durch einfaches axiales Aufschieben des Schneidenhalters 3 auf das Handstück 4 ermöglicht werden, da die beiden U-förmigen Ausnehmungen 45 im Handstück 2 mit ihren Wänden eine Führung der Verbindungsanordnung 4 an den Drückbereichen 42 ermöglichen. Weiterhin kann der Ring 46 mit den integral daran gebildeten Armen 40 auch vom Schneidenhalter 3 gelöst werden und somit Schneidenhalter 3 und Verbindungsanordnung 4 separat gereinigt werden.

### Bezugszeichenliste

- 1: medizinisches Instrument
- 2: Handstück
- 3: Schneidenhalter
- 4: Verbindungsanordnung
- 5: Tastenanordnung
- 6: Steuerplatine
- 20: Handstückkörper
- 21: Absaugkanal
- 22: Hohlraum im Handstückkörper
- 30: äußeres Hohlrohr
- 31: inneres Schneidenrohr
- 32: Schneiden
- 33: Austrittsöffnung
- 40: Arme
- 41: Rastelement
- 41a: Rampe
- 42: Drückbereich
- 43: elastischer Bereich
- 44: Aufnahme
- 44a: Hinterschneidung
- 45: U-förmige Ausnehmung
- 46: Ring
- 47: Clips-Verbindung
- 50: Tastenplatte
- 51: erste Taste
- 52: zweite Taste
- 53: dritte Taste
- 54: Absaugregulierung
- 55: erster Tastkontakt
- 56: zweiter Tastkontakt
- 57: Deckel
- 58: seitlicher Vorsprung
- 59: seitliche Aussparung im Deckel
- X-X: Axialrichtung
- Y-Y: Kippachse der Tastenanordnung

## Patentansprüche

1. Medizinisches Instrument für arthroskopische Eingriffe, umfassend:
- ein Handstück (2),
- ein Schneidenhalter (3) mit einem äußeren Hohlrohr (30) und einem inneren hohlen Schneidenrohr (31) mit Schneiden (32) an einem distalen Ende des Innenrohrs, und
- eine Verbindungsanordnung (4) zum lösbaren Verbinden des Handstücks (2) mit dem Schneidenhalter (3),
- wobei die Verbindungsanordnung (4) zwei sich in Axialrichtung (X-X) erstreckende Arme (40) am Schneidenhalter (3) aufweist, die sich einander gegenüberliegen und an ihren freien Enden ein Rastelement (41) aufweist, wobei die Arme (40) ferner jeweils einen Drückbereich (42) und einen elastischen Bereich (43) aufweisen,
- wobei das Handstück (2) an einem Innenumfang eine Aufnahme (44) mit einer Hinterschneidung (44a) für die Rastelemente (41) aufweist, und
- wobei das Handstück ferner an einem distalen Endbereich zwei Ausnehmungen (45) zur teilweisen Aufnahme der Drückbereiche (42) aufweist.

2. Instrument nach Anspruch 1, wobei die Drückbereiche (42) radial von den Armen (40) nach außen vorstehen.

3. Instrument nach einem der vorhergehenden Ansprüche, wobei die Rastelemente (41) jeweils eine Rampe (41a) aufweisen.

4. Instrument nach einem der vorhergehenden Ansprüche, wobei die Arme (40) einstückig an einem Ring (46) ausgebildet sind, wobei der elastische Bereich (43) der Arme (40) zwischen dem Ring (46) und den Drückbereichen (42) angeordnet ist.

5. Instrument nach Anspruch 4, wobei der Ring (46) mittels einer Clips-Verbindung (47) mit dem Schneidenhalter (3) verbunden ist.

6. Instrument nach einem der vorhergehenden Ansprüche, wobei die Ausnehmungen (45) im Handstück (2) U-förmig ausgebildet sind und die Drückbereiche (42) entsprechend den Ausnehmungen U-förmig ausgebildet sind.

7. Instrument nach einem der vorhergehenden Ansprüche, wobei am Handstück (2) eine Tastenanordnung (5) zur Betätigung des medizinischen Instruments nahe dem distalen Ende des Handstücks (2) angeordnet ist, wobei die Tastenanordnung (5) in Umfangsrichtung zwischen den Ausnehmungen (45) für die Aufnahme der Drückbereiche (42) der Verbindungsanordnung (4) angeordnet ist.

8. Instrument nach Anspruch 7, wobei die Tastenanordnung (5) eine erste Taste (51), eine zweite Taste (52) und eine dritte Taste (53) sowie einen ersten Tastkontakt (54) und einen zweiten Tastkontakt (55) aufweist, wobei die erste Taste (51) eingerichtet ist, den ersten Tastkontakt zu betätigen, die zweite Taste (52) eingerichtet ist, den zweiten Tastkontakt zu betätigen und die dritte Taste (53) eingerichtet ist, gleichzeitig den ersten und zweiten Tastkontakt zu betätigen.

9. Instrument nach Anspruch 8, wobei die drei Tasten (51, 52, 53) der Tastanordnung (5) in Reihe in Axialrichtung (X-X) derart angeordnet sind, dass die dritte Taste zwischen der ersten und zweiten Taste angeordnet ist und wobei die Tasten (51, 52, 53) auf einer gemeinsamen, starren Tastenplatte (50) angeordnet sind.

10. Instrument nach einem der Ansprüche 7 bis 9, ferner umfassend eine Absaugregulierung (54), welche in Reihe mit der Tastenanordnung (5) in Axialrichtung angeordnet ist.
